# EUROPEAN PATENT SPECIFICATION

(11) **EP 1 819 393 B1**
(45) Date of publication and mention of the grant of the patent: **06.10.2010**
(21) Application number: 05852855.5
(22) Date of filing: 05.12.2005
(51) Int. Cl.: A61M 37/00

(54) **MEDICAL DEVICE**
MEDIZINISCHE VORRICHTUNG
DISPOSITIF MEDICAL

(30) Priority: 10.12.2004 US 634905 P
(43) Date of publication of application: 22.08.2007
(73) Proprietor: 3M Innovative Properties Company, St. Paul, MN 55133-3427 (US)
(72) Inventor: CARTER, Chad J., Saint Paul, Minnesota 55133-3427 (US); FREDERICKSON, Franklyn L., Saint Paul, Minnesota 55133-3427 (US); HANSEN, Richard G.,, Saint Paul, Minnesota 55133-3427 (US); HART, John R.,, Saint Paul, Minnesota 55133-3427 (US); LANDIN, Donald T.,, Saint Paul, Minnesota 55133-3427 (US); TOKIE, Jeffrey H.,, Saint Paul, Minnesota 55133-3427 (US); WIRTANEN, David J.,, Saint Paul, Minnesota 55133-3427 (US)
(74) Representative: Vossius & Partner
(86) International application number: PCT/US2005/043769
(87) International publication number: WO 2006/062848

(56) References cited:
- WO-A-00/74763
- WO-A-03/059431
- GB-A- 2 221 394
- US-A1- 2003 045 837
- US-B1- 6 565 532
- US-B1- 6 656 147

## Description

The present invention relates to a medical device suitable for use in the delivery of active component into or through the skin.

Pharmaceutical compositions, vaccines, drugs, therapeutic substances, etc.. ("active components") may be delivered into the body through the skin in any of a number of different ways. The main barrier to the transport of therapeutic substances through the skin is the outermost layer of the skin known as the stratum corneum. To deliver a therapeutic substance through the skin, the molecule must be provided with a pathway through the stratum corneum. Active components, can be delivered through the skin by injection using a hypodermic syringe with a hollow needle to puncture the stratum corneum and deliver the active component beneath the skin. Other means for the delivery of certain therapeutic substances include transdermal patches, ointments or lotions as well as microneedle arrays.

Ointments or lotions can be formulated with an active component and a suitable biocompatible carrier so that, when applied to the skin, the active component can be delivered into the body by absorption through the stratum corneum. Transdermal adhesive patches are also available and are generally constructed as an adhesive article with a pressure sensitive adhesive coated onto the surface of a backing comprised of a polymeric film, cloth or the like. Transdermal adhesive patches are provided with an adhesive that allows the patch to be releasably adhered to the surface of the skin where a predetermined dosage of an active component can be put in contact with a small surface area of the skin. An appropriate biocompatible carrier is normally provided to facilitate the absorption of the therapeutic substance through the stratum corneum over a period of time while the patch remains adhered to the skin.

Microneedle arrays also provide a means for the delivery of active components through the skin. Microneedle arrays are devices that include a plurality of small piercing elements often referred to as microneedles, microneedle arrays, micro arrays, micro-pins or the like. The small piercing elements on these devices pierce the stratum corneum upon contact, making a plurality of microscopic slits which serve as passageways through which active components can be delivered into the body. In delivering an active component, the microneedle array can be provided with a reservoir for temporarily retaining an active component in liquid form prior to delivering the active component through the stratum corneum. In some constructions, the microneedles can be hollow to provide a liquid flow path directly from the reservoir and through the microneedles to enable delivery of the therapeutic, substance through the skin. In alternate constructions, active component(s) may be coated and dried on the microneedle array and delivered directly through the skin after the stratum corneum has been punctured. Additionally, microneedle devices can be provided as transdermal patches by providing the device in a construction that permits adhesive attachment of the microneedle array to the skin of a mammal. In still other constructions, microneedle devices permit the sampling of transdermal body analytes as they exit the body through the microscopic slits.

Microneedle devices such as the aforementioned patch may also be associated with an applicator device to assist in the placement of the microneedle device on the skin. In some constructions, the applicator can provides sufficient force during the application of the microneedle device to the skin so that the microneedles have a higher likelihood of effectively piercing the stratum corneum.

U.S. Patent 6,565,532 describes a microneedle apparatus used for marking skin and for dispensing semi-permanent subcutaneous makeup.

G.B. Patent 2,221,394 describes an injection device for delivering a liquid using microneedle delivery.

U.S. Patent 6,656,147 is directed to a device for the transdermal delivery of a substance, such as a drug or vaccine or other pharmaceutical agent (or active agent), to a patient where the active agent is carried through the stratum corneum by a carrier liquid. The active agent is reconstituted, dispersed or carried through the stratum corneum by the puncturing of a bladder in the device.

WO 03/059431 describes microneedle devices and methods of manufacturing microneedle devices where the devices have a capillary volume in fluid communication with the base of each microneedle in the device. The devices described therein may be used for delivering medicaments or substances and/or extracting blood or tissue from a patient. Manufacturing methods may include simultaneous application of pressure and ultrasonic energy when piercing a cover which, in combination with the substrate, defines the capillary volume.

The present invention provides a medical device suitable for use in the delivery of active component into or through the skin in accordance with claim 1.

Those skilled in the art will better understand the features of the invention upon consideration of the remainder of the disclosure, including the various figures as described in the detailed description and the appended claims.

In describing embodiments of the invention herein, reference is made to the various
Figures in which like reference numerals indicate like structures and wherein:
Figure 1 is a photomicrograph of a microneedle array suitable for use in the present invention;
Figure 2 is a perspective view of a microarray patch device;
Figure 3 is a side elevation, in cross section, of the microarray patch device of Figure 2;
Figure 4 is a side elevation, in cross section, of another microarray patch device;
Figure 5 is a side elevation, in cross section, of still another microarray patch device;
Figure 6 is a side elevation, in cross section, of still another microarray patch device;
Figure 7 is a perspective view of an embodiment of a microarray patch device according to the present invention;
Figure 8 is a side elevation, in cross section, of the microarray patch device of Figure 7;
Figure 9 is a side elevation, in cross section, of another embodiment of a microarray patch device according to the present invention;
Figure 10 is a side elevation, in cross section, of still another embodiment of a microarray patch device according to the present invention; and
Figure 11 is a side elevation, in cross section, of still another embodiment of a microarray patch device according to the present invention.

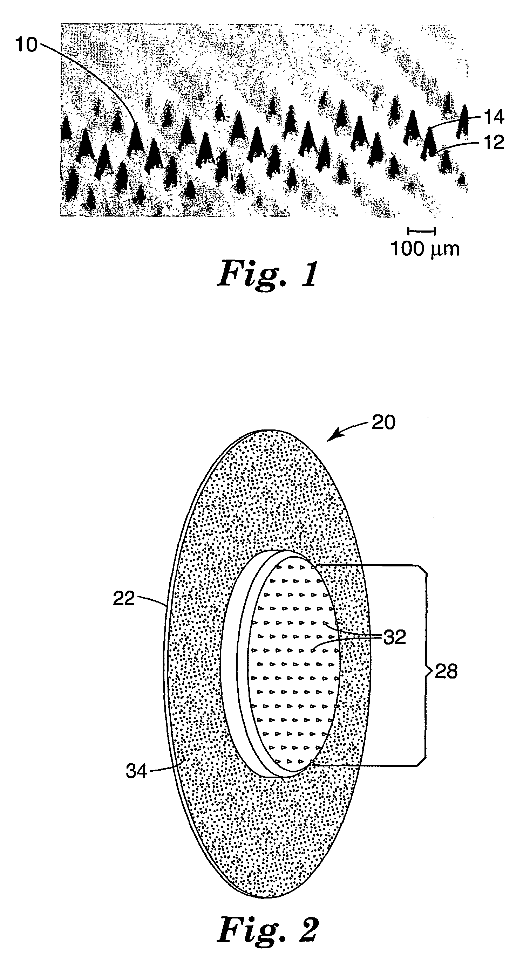

The invention provides a device having a microneedle array and which can be affixed to the skin to facilitate the delivery of active components into or through mammalian skin. Referring to the various Figures, a microneedle array suitable for use in the present invention is illustrated in Figure 1. The general shape of the microneedle 10 is a tapered projection having a larger base 12 tapering to a narrow tip 14 which is generally able to pierce mammalian skin. Therapeutic substances such as vaccine or a pharmacologically active material may be applied (e.g., by coating) to the outer surfaces of the microneedles 10 to deliver the substance to a patient when the microneedles 10 pierce the stratum corneum of the patient's skin. As shown, the microneedles 10 can be arranged in uniformly spaced rows. In some embodiments, arrays of microneedles used in the present invention can have a distal-facing surface area of more than about 0.1 cm² and less than about 20 cm², and typically more than about 0.5 cm² and less than about 5 cm².

In the array shown in Figure 1, a portion of the surface from which the microneedles 10 project may be non-patterned in that the portion of the surface is free of microneedles. In one example the non-patterned surface has an area of more than about 1 percent and less than about 75 percent of the total area of the device surface that faces a skin surface of a patient. In one example the non-patterned surface has an area of more than about 0.10 square inch (0.65 cm²) to less than about 1 square inch (6.5 cm²). In another example (not shown), the microneedles can be disposed over substantially the entire surface area of the array 22.

While the illustrated microneedles 10 are depicted as spiked projections extending in uniform rows from a surface, it will be appreciated that the actual shape of the individual microneedles used in devices of the invention may be selected from any of a variety of shapes including without limitation pyramidal, conical or the like. One suitable configuration for the microneedle arrays includes the structures disclosed in United States patent application publication no. US2003/0045837 which describes microstructures in the form of microneedles having tapered structures that include at least one channel formed in the outside surface of each microneedle. Where the microneedles have bases that are elongated in one direction, the channels can extend from one of the ends of each elongate base to the tips of the microneedles. Optionally, the aforementioned channels can be terminated short of the tips of the microneedles. The microneedle arrays may also include conduit structures formed on the surface of the substrate on which the microneedle array is located, and the aforementioned channels can be constructed to permit fluid communication with the conduit structures.

Suitable microneedles may have generally vertical wall angles, i.e. the microneedles may be in the form of pins, with sidewalls that are largely orthogonal to the surface of the substrate from which they protrude.

Suitable microneedles for use in devices of the present invention may also be characterized by their aspect ratio. As used herein, the term "aspect ratio" refers to the ratio of the height of the microneedle (above the surface surrounding the base of the microneedle) to the maximum base dimension, that is, the longest straight-line dimension that the base occupies (on the surface occupied by the base of the microneedle). In embodiments of the present invention, the microneedles can have an aspect ratio of 2:1 or higher. In some embodiments, the microneedles can have an aspect ratio of 3:1 or higher.

Still another suitable microneedle construction comprises the structures described in United States Patent No. 6,091,975 (Daddona, et al.) which describes blade-like microprotrusions for piercing the skin. Still another microneedle construction comprises the structures described in United States Patent No. 6,312,612 (Sherman, et al.) which describes tapered structures having a hollow central channel. Still another suitable microneedle construction comprises structures like those described in International Publication No. WO 00/74766 (Gartstein, et al.) which describes hollow microneedles having at least one longitudinal blade at the top surface of tip of the microneedle. Another suitable microneedle construction includes a generally conical shape wherein the microneedles may have a defined tip bluntness, like those described in co-pending and commonly owned U.S. Patent Application Serial No. 10/621620, filed on July 17, 2003, which describes microneedles have a flat tip comprising a surface area measured in a plane aligned with the base of about 20 square micrometers or more and 100 square micrometers or less. In some embodiments, the surface area of the flat tip is measured as the cross-sectional area measured in a plane aligned with the base, the plane being located at a distance of 0.98 or 98% of the height of the microneedle above the substrate surface measured from base to tip.

In some embodiments, the microneedles are provided as a single array comprising a multitude of individual microneedles which are manufactured integrally with the device of the invention. In some embodiments, the microneedles can initially be provided separately and later added to the substrate during the manufacture or assembly of the device.

The microneedles may be manufactured from any of a variety of materials, and the actual material selected for a particular microneedle array can be based on a variety of factors including the ability of the material to accurately reproduce the desired microneedle pattern; the strength and toughness of a particular material when formed into the microneedles; the compatibility of a material with mammalian skin; the compatibility of a material with body fluids expected to contact the microneedle array, etc.

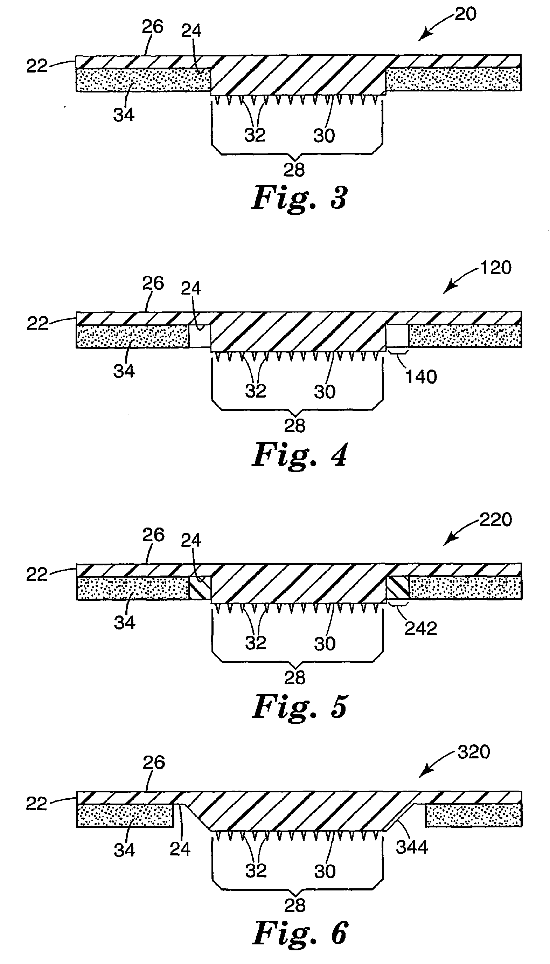

Referring to Figures 2 and 3, a patch 20 without a flexible backing member is depicted. The patch 20 includes an extension member 22 with a first major surface 24 and a second major surface 26. The first major surface 24 of the extension member 22 comprises a first portion having an array retaining member 28 extending therefrom. The array retaining member 28 includes an array surface 30 having at least one microneedle 32 extending from the surface 30. A second portion of the first major surface 24 of extension member 22 includes a layer of pressure sensitive adhesive 34 disposed thereon. The pressure sensitive adhesive is provided on the second portion of the surface 24 of the extension member 22 to facilitate the adhesive attachment of the device 20 to mammalian skin when the at least one microneedle 32 is inserted through the stratum corneum. In some embodiments, the layer of adhesive 34 is provided at a thickness that keeps the adhesive layer 34 from extending beyond the surface 30 of the array retaining member 28. In some embodiments, the adhesive layer 34 will extend from the first major surface 24 of the extension member 22 to a height less than the height of the array surface 30.

While the patch 20 is depicted essentially in a circular configuration with the extension member 22 surrounding the array retaining member 28, it will be appreciated that the patch 20 may be configured in any useful or ornamental configuration desired. Moreover, the extension member 22 may be dimensioned to extend from but not necessarily surround the entire array retaining member 28. Similarly, the array retaining member may be configured in a different geometric shape than the circular configuration depicted in Figure 2. It will further be appreciated that the description herein of the various embodiments of the invention are merely exemplary of patches that embody the principles of the present invention and that the described embodiments are not intended to be limitation on the broader concepts inherent in the described embodiments.

Another patch 120 without a flexible backing member is shown in Figure 4. The patch 120 is constructed essentially in the same manner as patch 20 shown in Figures 2 and 3 and described above. However, patch 120 includes a gap 140 between the pressure sensitive adhesive layer 34 and the array retaining member 28. Construction of the patch 120 will require less adhesive than the patch 20 of Figures 2 and 3. Moreover, gap 140 provides a small buffer to minimize the potential for the adhesive 34 to spread or migrate closer to the array retaining member 28 and the microneedles 32.

Still another patch 220 without a flexible backing member is depicted in Figure 5. The patch 220 is essentially of the same construction as the patch 120 except that the patch 220 includes a barrier member 242 extending between the adhesive layer 34 and the array retaining member 28. The barrier member 242 is dimensioned to enhance and maintain the separation between the adhesive 34 and the microneedles 32 of the array retaining member 28. The barrier member 242 may comprise a film, polymer or other inert material and desirably will isolate the array retaining member 28 from the adhesive 34 to inhibit and prevent significant migration of material between adhesive 34 and any therapeutic substances coated on the microneedles 32.

Another patch 320 without a flexible backing member is depicted in Figure 6. The patch 320 is constructed substantially as described with respect to the patch 120 shown in Figure 4, except that the array retaining member 28 includes sloping sides 344 that function as a barrier or buffer between the adhesive layer 34 and the array retaining member 28 to enhance and maintain the separation between the adhesive 34 and any the microneedles 32 of the array retaining member 28.

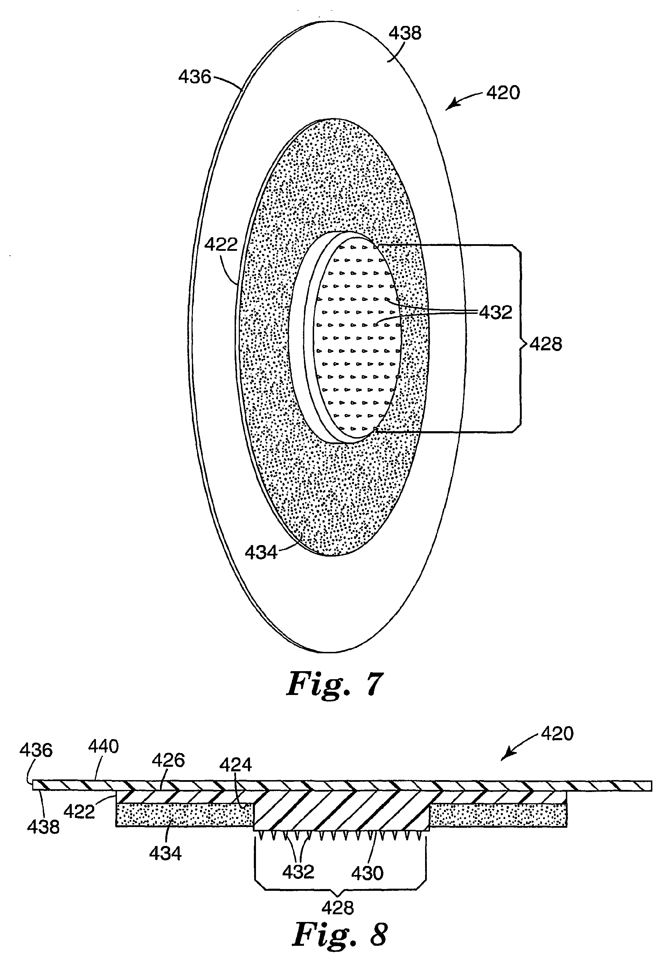

Referring now to Figures 7 and 8, an embodiment of a patch 420 is shown according to the invention. The patch 420 includes an extension member 422 with a first major surface 424 and a second major surface 426. The first major surface 424 of the extension member 422 comprises a first portion having, as an integral part thereof, an array retaining member 428 extending therefrom. The array retaining member 428 includes an array surface 430 having at least one microneedle 432 extending from the surface 430. A second portion of the first major surface 424 of extension member 422 includes a layer of pressure sensitive adhesive 434 disposed thereon. The pressure sensitive adhesive is provided on the second portion of the surface 424 of the extension member 422 to facilitate the adhesive attachment of the device 20 to mammalian skin when the at least one microneedle 432 is inserted through the stratum corneum. In some embodiments, the layer of adhesive 434 is provided at a thickness that keeps the adhesive layer 434 from extending beyond the surface 430 of the array retaining member 428. In some embodiments, the adhesive layer 434 will extend from the first major surface 424 of the extension member 422 to a height less than the height of the array surface 430.

The patch 420 additionally includes a flexible backing member 436 having a first major surface 438 and a second major surface 440. The first major surface 438 of the flexible backing member 436 is affixed (e.g., adhesively) to the second major surface 426 of the extension member 422. A portion of the flexible backing member 436 extends beyond the outer edge of the extension member 422. In this arrangement of parts, the flexible backing member 436 may be used for securing the patch 420 to the skin of a patient. In this regard, the first major surface 438 of the backing member 436 will typically contact the skin of the patient and be secured thereto be any of a variety of suitable means such as, for example, medical grade adhesive tape or the like (not shown). In this embodiment, the first major surface 438 covers a portion of the patient's skin and can serve to enlarge the zone around the array retaining member 428 that is created by the first major surface 424 of the extension member 422 to assure that the patch 420 remains in place for the desired amount of time and to assist in keeping dirt or other contaminants away from the punctures in the stratum corneum created by the microneedles 432. In all other respects, the patch 420 operates in essentially the same manner as described in the foregoing embodiments.

While the patch 420 is depicted essentially in a circular configuration with the extension member 422 surrounding the array retaining member 428, it will be appreciated that the patch 420 may be configured in any useful or ornamental configuration desired. Moreover, the extension member 422 may be dimensioned to extend from but not necessarily surround the entire array retaining member 428. Similarly, the array retaining member may be configured in a different geometric shape than the circular configuration depicted in Figures 7 and 8. Finally, the flexible backing member 436 can be of any desired shape, size or configuration.

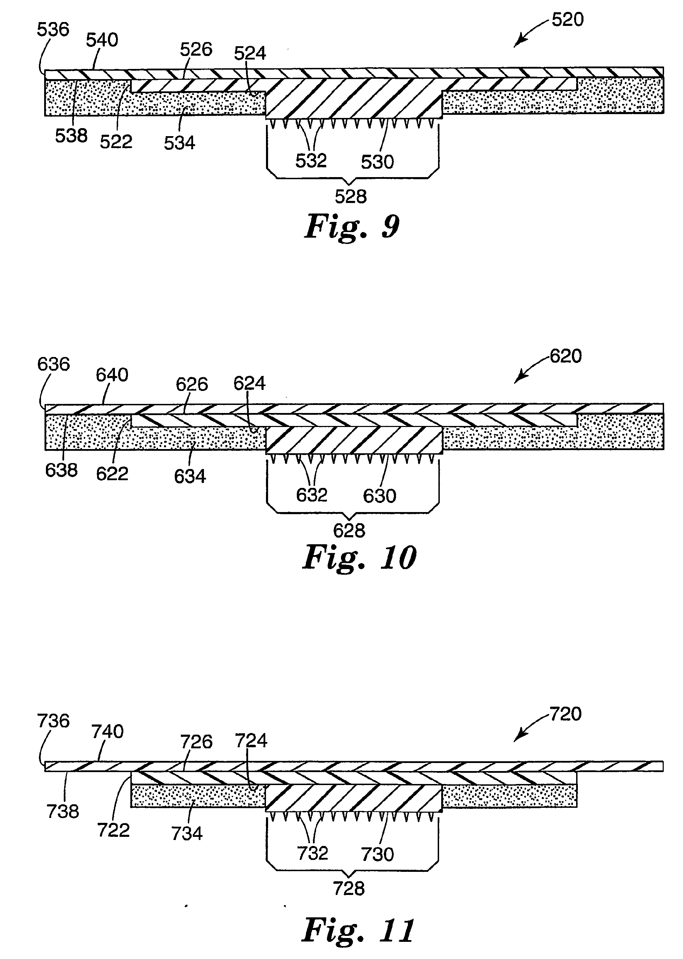

Still another embodiment is illustrated in Figure 9. A patch 520 is provided and includes an extension member 522 with a first major surface 524 and a second major surface 526. The first major surface 524 of the extension member 522 comprises a first portion having, as an integral part thereof, an array retaining member 528 extending therefrom. The array retaining member 528 includes an array surface 530 having at least one microneedle 532 extending from the surface 530. A second portion of the first major surface 524 of extension member 522 includes a layer of pressure sensitive adhesive 534 disposed thereon. The pressure sensitive adhesive is provided on the second portion of the surface 524 of the extension member 522 to facilitate the adhesive attachment of the device 520 to mammalian skin when the at least one microneedle 532 is inserted through the stratum corneum. In some embodiments, the layer of adhesive 534 is provided at a thickness that keeps the adhesive layer 534 from extending onto the surface 530 of the array retaining member 528. In some embodiments, the adhesive layer 534 will extend from the first major surface 524 of the extension member 522 to a height less than the height of the array surface 530.

The patch 520 additionally includes a flexible backing member 536 having a first major surface 538 and a second major surface 540. The first major surface 538 of the flexible backing member 536 is affixed (e.g., adhesively) to the second major surface 526 of the extension member 522. A portion of the flexible backing member 536 extends beyond the outer edge of the extension member 522 and adhesive layer 534 likewise is extended to cover at least a portion of the first major surface 538 of flexible backing member 536 so that the flexible backing layer 536 is equipped to assist in securing the patch 520 to the skin of a patient. In this regard, the adhesive layer 534 on both the first major surface 538 of the backing member 536 and on the first major surface 524 of the extension member 522 will typically contact and secure the skin of the patient to the patch 520. First major surface 538 serves to enlarge the zone around the array retaining member 528 that is created by the first major surface 524 of the extension member 522 to assure that the patch 520 remains in place and to assist in keeping dirt or other contaminants away from the punctures in the stratum corneum created by the microneedles 532. In all other respects, the patch 520 operates in essentially the same manner as described in the foregoing embodiments.

It will be appreciated that the patch 520 may be configured in any useful or ornamental configuration desired and is not limited to a circular configuration. Moreover, the extension member 522 may be dimensioned to extend from but not necessarily surround the entire array retaining member 528. Similarly, the array retaining member 528 may be configured in a different geometric shape than the circular configuration depicted in Figure 9. Finally, the flexible backing member 536 can be of any desired shape, size or configuration.

Still another embodiment of the invention is illustrated in Figure 10. A patch 620 is provided and includes an extension member 622 with a first major surface 624 and a second major surface 626. The first major surface 624 of the extension member 622 comprises a first portion that is affixed to an array retaining member 628. In this embodiment, the extension member 622 is affixed to but is apart and distinct from the array retaining member 628. The extension member 622 is affixed to the array retaining member 628 by any suitable means including by use of a suitable adhesive, or by heat or melt bonding, and the like.

The array retaining member 628 includes an array surface 630 having at least one microneedle 632 extending from the surface 630. A second portion of the first major surface 624 of extension member 622 includes a layer of pressure sensitive adhesive 634 disposed thereon. The pressure sensitive adhesive is provided on the second portion of the surface 624 of the extension member 622 to facilitate the adhesive attachment of the device 620 to mammalian skin when the at least one microneedle 632 is inserted through the stratum corneum. In some embodiments, the layer of adhesive 634 is provided at a thickness that keeps the adhesive layer 634 from extending onto the surface 630 of the array retaining member 628. In some embodiments, the adhesive layer 634 will extend from the first major surface 624 of the extension member 622 to a height less than the height of the array surface 630.

The patch 620 additionally includes a flexible backing member 636 having a first major surface 638 and a second major surface 640. The first major surface 638 of the flexible backing member 636 is affixed (e.g., adhesively) to the second major surface 626 of the extension member 622. A portion of the flexible backing member 636 extends beyond the outer edge of the extension member 622 and adhesive layer 634 likewise is extended to cover at least a portion of the first major surface 638 of flexible backing member 636 so that the flexible backing layer 636 is equipped to assist in securing the patch 620 to the skin of a patient. In this regard, the adhesive layer 634 on both the first major surface 638 of the backing member 636 and on the first major surface 624 of the extension member 622 will typically contact secure the skin of the patient to the patch 620. First major surface 638 serves to enlarge the zone around the array retaining member 628 that is created by the first major surface 624 of the extension member 622 to assure that the patch 620 remains in place and to assist in keeping dirt or other contaminants away from the punctures in the stratum corneum created by the microneedles 632. In all other respects, the patch 620 operates in essentially the same manner as described in the foregoing embodiments.

It will be appreciated that the patch 620 may be configured in any useful or ornamental configuration. Moreover, the extension member 622 may be dimensioned to extend from but not necessarily surround the entire array retaining member 628. Similarly, the array retaining member 628 may be configured in a different geometric shape than the circular configuration depicted in Figure 10. Finally, the flexible backing member 636 can be of any desired shape, size or configuration.

Referring now to Figure 11, another embodiment of a patch 720 is shown according to the invention. The patch 720 includes an extension member 722 with a first major surface 724 and a second major surface 726. In this embodiment, the extension member 722 is apart and distinct from the array retaining member 728. The extension member 722 may be affixed to the array retaining member 728 by any suitable means including by use of a suitable adhesive, heat or melt bonding, and the like.

The array retaining member 728 includes an array surface 730 having at least one microneedle 732 extending from the surface 730. A second portion of the first major surface 724 of extension member 722 includes a layer of pressure sensitive adhesive 734 disposed thereon. The pressure sensitive adhesive is provided on the second portion of the surface 724 of the extension member 722 to facilitate the adhesive attachment of the device 720 to mammalian skin when the at least one microneedle 732 is inserted through the stratum corneum. In some embodiments, the layer of adhesive 734 is provided at a thickness that keeps the adhesive layer 734 from extending beyond the surface 730 of the array retaining member 728. In some embodiments, the adhesive layer 734 will extend from the first major surface 724 of the extension member 722 to a height less than the height of the array surface 730.

The patch 720 additionally includes a flexible backing member 736 having a first major surface 738 and a second major surface 740. The first major surface 738 of the flexible backing member 736 is affixed (e.g., adhesively) to the second major surface 726 of the extension member 722. A portion of the flexible backing member 736 extends beyond the outer edge of the extension member 722. In this arrangement of parts, the flexible backing member 736 may be used for securing the patch 720 to the skin of a patient. In this regard, the first major surface 738 of the backing member 736 will typically contact the skin of the patient and be secured thereto be any of a variety of suitable means such as, for example, medical grade adhesive tape or the like (not shown). In this embodiment, the first major surface 738 covers the a portion of the patient's skin and can serve to enlarge the zone around the array retaining member 728 that is created by the first major surface 724 of the extension member 722 to assure that the patch 720 remains in place for the desired amount of time and to assist in keeping dirt or other contaminants away from the punctures in the stratum corneum created by the microneedles 732. In all other respects, the patch 720 operates in essentially the same manner as described in the foregoing embodiments.

While the patch 720 is depicted essentially in a circular configuration with the extension member 722 surrounding the array retaining member 728, it will be appreciated that the patch 720 may be configured in any useful or ornamental configuration desired. Moreover, the extension member 722 may be dimensioned to extend from but not necessarily surround the entire array retaining member 728. Similarly, the array retaining member may be configured in a different geometric shape than the circular configuration depicted in Figure 11. Finally, the flexible backing member 736 can be of any desired shape, size or configuration.

In the foregoing embodiments, the flexible backing member may comprise any of a variety of materials. In some embodiments, the flexible backing member will comprise a material selected from polypropylene; polycarbonate; polyethylene, particularly low density polyethylene, linear low density polyethylene, metallocene polyethylenes, and high density polyethylene; polyvinyl chloride; polyester (e.g., polyethylene terephthalate); polyvinylidene chloride; ethylene-vinyl acetate (EVA) copolymer; polyurethane; cellulose acetate; and ethyl cellulose. Coextruded multilayer polymeric films are also suitable, such as those described in U. S. Patent No. 5,783,269 (Heilmann et al.). Backings that are layered such as polyethylene terephthalate-aluminum-polyethylene composites and polyethylene terephthalate-EVA composites are also suitable. Foam tape backings, such as closed cell polyolefin films used in 3M™ 1777 Foam Tape and 3M™ 1779 Foam Tape are also suitable. Fabrics and non-wovens are likewise suitable. In some embodiments, the flexible backing member is a polymer film made of polyethylene terephthalate, polycarbonate, or polyethylene. In other embodiments, the flexible backing member is a polyethylene terephthalate polymer film.

It will be appreciated that the features described in connection with an embodiment herein, may be used in other embodiments, and the various features described in each of the embodiments may also be varied while still remaining within the scope of the invention. For example, the pressure sensitive adhesive utilized in each of the embodiments of Figures 7 through 11 may be present in any of a variety of patterns. For example, the adhesive layers (e.g., adhesive layers 534, 634) may be patterned or non-patterned, and may be continuous or discontinuous. The adhesive layer may additionally be interrupted by spaces, gaps or structures such as the gap 140 of Figure 4 or the barrier member 242 shown in Figure 5. The array retaining member may be provided in any of a variety of configurations and may include sloping sides similar or identical to the sides 344 of the array retaining member 28 (Figure 6), or the like. In general, the invention is intended to include any and all variations on the structures depicted in the various embodiments described above.

As described in connection with the various embodiments, the present invention provides a medical device in the form of a patch for the delivery of an active component through the stratum corneum. In some embodiments, the patch is constructed from a single molded polymeric material. In some embodiments, the patch is provided as a one-piece molded article wherein the extension member, array retaining member and the microneedle array (as generally described herein) are molded as a single piece from the same material(s). Suitable materials for these one-piece articles include those selected from materials such as acrylonitrile-butadiene-styrene (ABS) polymers, polyphenyl sulfides, polycarbonates, polypropylenes, acetals, acrylics, polyetherimides, polybutylene terephthalates, polyethylene terephthalates as well as other known materials and combinations of two or more of the foregoing. A suitable method for molding the microarrays of the invention is described in co-pending patent application serial no. 60/546780 filed February 23, 2004. It will be appreciated that a patch according to the present invention should be sufficiently flexible to allow for uniform adhesion of the extension member to the area of mammalian skin to which the patch is applied. Moreover, the surface of the extension member will provide a surface area sufficient to uniformly adhere the patch to an area of mammalian skin to permit the effective delivery of an active component over a period of time.

In some of the embodiments that comprise a flexible backing member, the extension member, array retaining member and the microneedle array may be molded as a single piece from the same materials described herein. In other embodiments that comprise a flexible backing member, the extension member, array retaining member and the microneedle array may be provided as separate parts which may or may not be molded and may or may not be of the same material(s). In embodiments where the extension member and the array retaining member are provided as separate parts, they are typically affixed to one another, such as by a suitable adhesive or by melt bonding, for example.

The medical device of the invention is designed to permit its application to the surface of mammalian skin using an applicator or other means for the delivery of the medical device with sufficient force to pierce the stratum corneum as well as adhere the extension member to the surface of the skin. Accordingly, the second major surface of the medical device is normally provided as a relatively smooth and featureless surface so that a force may be applied to the medical device uniformly along the second surface in order to affix it to the desired portion of the skin. Hence, the second surface of the extension member will not normally include additional structures such as, for example, a reservoir for the retention or temporary storage of active component in liquid form.

The devices of the invention can be used as transdermal patches in methods for the delivery of one or more active materials through mammalian skin by providing the microneedles in a construction that facilitates penetration of the stratum corneum. In some embodiments, a medicament or therapeutic agent may be applied directly to an area of the skin, and thereafter the microneedle array can be applied with suitable force to the same area of the skin to puncture the stratum corneum and allow the therapeutic agent to enter the body through the punctures made by the individual microneedles. In other embodiments, the active component may first be applied directly to the microstructured area of the array (e.g., as a coating). In some embodiments, the active component may be applied to the microneedle array when the active component is a liquid or is dissolved in a liquid or is suspended within a liquid as a suspension or colloid. After application to the microneedle array, the active component may be dried prior to applying it to mammalian skin. Alternatively, the active component may be applied to mammalian skin while the active component still comprises a liquid. The microneedle array, coated with active component, can be applied to the skin with force sufficient to puncture the stratum corneum. The active component coated on the microstructured area of the array will be mechanically deposited into the skin tissue or it may be dissolved from the array by body fluids, thus allowing the therapeutic agent or medicament to be absorbed into the skin tissue. The parameters for the delivery of therapeutic agents using the medical devices of the invention are suitably described in the aforementioned co-pending patent applications, serial nos. 09/947195 (publication no. US2003/0045837) and 10/621620. A suitable method of use is described in conjunction with the applicator disclosed in U.S. Provisional Patent Application No. 60/578651 filed on June 10, 2004 (atty. Ref. 59403US002).

### Examples

### Microneedle arrays

Microneedle arrays were prepared as follows. A circular disk (area 2 cm², thickness 1.02 mm) that was partially patterned with an array of microneedles (37 x 37) in a square shape (1 cm²) centered on one side of the disk was prepared. The needles were regularly spaced with a distance of 275 microns between the tips of adjacent needles in a square-shaped pattern. Individual needles were pyramidal in shape with a height of 250 microns and a square base having a side-length of 83.3 microns. The tips were truncated with a flat, square-shaped top having a side-length of 5 microns. Arrays were injection molded according to the general description provided in International Patent Application Publication No. WO 05/82596 and made from polycarbonate (Lexan® HPS1R-1125, GE Plastics, Pittsfield, MA). The center of the disk was then die cut to provide a microneedle array (area = 1 cm²) having microneedles on approximately 90% of the surface of the patterned side of the disk. The microneedle array had approximately 1200 microneedles.

### Delivery of patches from a storage collar

Microarray patches, as described below, were placed in a cylindrical storage collar, described in further detail in United States Patent Application No. 60/578651 and International Patent Application WO 05/020283, having small tabs on the inner surface of the cylinder for supporting the patch. A handheld, spring-driven patch applicator, as described in the aforementioned patent applications, was used to propel the patch from the storage collar. The applicator used a 2.88 gram piston that reached a maximum velocity of 7.2 m/s when triggered in the absence of a medical device. (M9 applicator) Velocity of the piston in the absence of a microarray patch was measured by placing a small piece of a matte-finish reflective tape on the outer face of the piston for purposes of conducting the velocity/displacement measurement. The applicator was placed against a fixture attached to a laser measuring device (Laser Vibrometer Controller model no. OFV-3001 and Laser Fiber Interferometer model no. OFV-502, Polytec Inc., Tustin, California) and aligned such that the laser could reflect off of the matte-finish reflective tape. Measurement of the velocity of a microarray patch from a storage collar was performed by replacing the patterned microarray with a "blank" array having the same physical dimensions and having a piece of matte-finish reflective tape applied to the blank in place of the microneedle patterning. The values reported below are the maximum velocity achieved by the microarray patch.

### Example 1

A microarray patch was constructed as follows. A ring of skin-contacting adhesive was formed from a three-layer laminate of double-sided tape (3M Transparent Polyester, 3.4 mil Double Coated Medical Tape 1513, 25.4 micron thick polyester film with 30.5 micron thick adhesive on each side) which was die cut into a ring having an outer diameter of 2.35 cm and an inner diameter of 1.22 cm. This ring was adhered to the outer rim of a circular piece (area = 2.5 cm²) of 10 mil (254 micron) thick polycarbonate film to form a first laminate.

A circular piece of double-sided tape (3M Transparent Polyester, 3.4 mil Double Coated Medical Tape 1513) with an area of 5.5 cm was adhered to a circular piece (area = 5.5 cm²) of polyethylene terephalate film with a thickness of 0.56 mil (14.2 micron) to form a second laminate.

The exposed side of the double-coated tape of the second laminate was adhered to the side of the polycarbonate film in the first laminate opposed to the skin-contacting adhesive. A circular piece (area = 1.0 cm²) of double-sided tape (3M Transparent Polyester, 3.4 mil Double Coated Medical Tape 1513) was used to adhere the non-patterned side of a microneedle array to the exposed area at the center of the polycarbonate film to make a finished microarray patch. All pieces described above were aligned concentrically. The microarray patch was placed in a storage collar as described above and the maximum velocity at which the device was propelled from the collar was 5.7 m/s.

### Example 2

A microarray patch was constructed as follows. A circular piece (area = 4.5 cm²) of skin-contacting adhesive was formed from a three-layer laminate of double-sided tape (3M Transparent Polyester, 3.4 mil Double Coated Medical Tape 1513). This piece was adhered to a circular piece (area = 4.5 cm²) of 10 mil (254 micron) thick polycarbonate film. The exposed side of the polycarbonate film was adhered to a second, circular piece (area = 4.5 cm²) of double-sided tape (3M Transparent Polyester, 3.4 mil Double Coated Medical Tape 1513). The exposed side of the second piece of double-coated tape was adhered to a circular piece (area = 5.5 cm²) of polyethylene terephalate film backing with a thickness of 2.0 mil (50.8 micron). The non-patterned side of a microneedle array was then adhered to the center of the exposed skin-contacting adhesive to make a finished microarray patch. All pieces described above were aligned concentrically. The microarray patch was placed in a storage collar as described above and the maximum velocity at which the device was propelled from the collar was 6.0 m/s.

### Example 3

A microarray patch was constructed as described in Example 2 with the exception that the polyethylene terephalate film backing had a thickness of 3.0 mil (76.2 micron). The microarray patch was placed in a storage collar as described above and the maximum velocity at which the device was propelled from the collar was 5.5 m/s.

## Claims

1. A medical device suitable for use in the delivery of active component into or through the skin, comprising:
an extension member (422; 522; 622; 722) having a first major surface (424; 524; 624; 724) and a second major surface (426; 526; 626; 726), the first major surface comprising a first portion and a flexible second portion;
an array retaining member (428; 528; 628; 728) extending from the first portion of the first major surface of the extension member, the array retaining member comprising an array surface (430; 530; 630; 730) having at least one microneedle (432; 532; 632; 732) extending from the array surface;
pressure sensitive adhesive (434; 534; 634; 734) disposed on the second portion of the first major surface of the extension member to facilitate the adhesive attachment of the device to mammalian skin when the at least one microneedle is inserted through the stratum corneum; and
a flexible backing member (436; 536; 636; 736) having a first major surface (438; 538; 638; 738) and a second major surface (440; 540; 640; 740), at least a portion of the first major surface of the flexible backing member being affixed to the second major surface of the extension member, the flexible backing member extending beyond the outer edge of the extension member.
**characterized in that** the second major surface of the extension member is relatively smooth and featureless.

2. The medical device of claim 1 wherein the extension member (422; 522; 622; 722) and the array retaining member (428; 528; 628; 728) comprise a one-piece construction comprising a material selected from the group consisting of acrylonitrile-butadiene-styrene (ABS) polymers, polyphenyl sulfides, polycarbonates, polypropylenes, acetals, acrylics, polyetherimides, polybutylene terephthalates, polyethylene terephthalates and combinations of two or more of the foregoing.

3. The medical device of claim 1 wherein the extension member and the array retaining member comprise polycarbonate.

4. The medical device of claim 1 wherein the extension member and the array retaining member are adhesively adhered to one another.

5. The medical device of claim 1, wherein the array surface (430; 530; 630; 730) comprises a plurality of identically configured microneedles (432; 532; 632; 732) extending from the array surface.

6. The medical device of claim 5, further comprising an active component retained on at least a portion of the identically configured microneedles.

7. The medical device of claim 1, wherein the array surface comprises a plurality of microneedles extending from the array surface.

8. The medical device of claim 7 wherein the microneedles comprise tapered structures that include at least one channel formed in the outside surface of each microneedle.

9. The medical device of claim 8 wherein the microneedles comprise elongate bases and the at least one channel in each microneedle extends from one of the ends of each elongate base to the tips of the microneedles.

10. The medical device of claim 7 wherein the microneedles have an aspect ratio of 2:1 or higher.

11. The medical device of claim 1, wherein the flexible backing member (436; 536; 636; 736) comprises a material selected from the group consisting of polyethylene terephthalate, polycarbonate, and polyethylene.

12. The medical device of claim 1 further comprising a pressure sensitive adhesive layer covering at least a portion of the first major surface of the flexible backing member.

## Patentansprüche

1. Medizinische Einrichtung, geeignet zur Verwendung bei der Lieferung einer aktiven Komponente in die oder durch die Haut, umfassend:
ein Verlängerungsglied (422; 522; 622; 722) mit einer ersten Hauptfläche (424; 524; 624; 724) und einer zweiten Hauptfläche (426; 526; 626; 726), wobei die erste Hauptfläche einen ersten Abschnitt und einen flexiblen zweiten Abschnitt umfasst;
ein Arrayhalterungsglied (428; 528; 628; 728), das sich von dem ersten Abschnitt der ersten Hauptfläche des Verlängerungsglieds erstreckt, wobei das Arrayhalterungsglied eine Arrayfläche (430; 530; 630; 730) mit mindestens einer Mikronadel (432; 532; 632; 732) umfasst, die sich von der Arrayfläche erstreckt;
Haftklebstoff (434; 534; 634; 734), der auf dem zweiten Abschnitt der ersten Hauptfläche des Verlängerungsglieds angeordnet ist, zum Erleichtern der Klebbefestigung der Einrichtung an der Haut eines Säugetiers, wenn die mindestens eine Mikronadel durch die Hornschicht eingeschoben ist; und
ein flexibles Trägerglied (436; 536; 636; 736) mit einer ersten Hauptfläche (438; 538; 638; 738) und einer zweiten Hauptfläche (440; 540; 640; 740), wobei mindestens ein Abschnitt der ersten Hauptfläche des flexiblen Trägerglieds an der zweiten Hauptfläche des Verlängerungsglieds befestigt ist, wobei sich das flexible Trägerglied über den Außenrand des Verlängerungsglieds hinaus erstreckt;
**dadurch gekennzeichnet, dass** die zweite Hauptfläche des Verlängerungsglieds relativ glatt und merkmalslos ist.

2. Medizinische Einrichtung nach Anspruch 1, wobei das Verlängerungsglied (422; 522; 622; 722) und das Arrayhalterungsglied (428; 528; 628; 728) eine einteilige Konstruktion umfassen, umfassend ein Material ausgewählt aus der Gruppe bestehend aus Acrylnitril-Butadien-Styrol-Polymeren (ABS -Polymeren), Polyphenylsulfiden, Polycarbonaten, Polypropylenen, Acetalen, Acrylen, Polyetherimiden, Polybutylenterephthalaten, Polyethylenterephthalaten und Kombinationen von zwei oder mehreren der obengenannten.

3. Medizinische Einrichtung nach Anspruch 1, wobei das Verlängerungsglied und das Arrayhalterungsglied Polycarbonat umfassen.

4. Medizinische Einrichtung nach Anspruch 1, wobei das Verlängerungsglied und das Arrayhalterungsglied aneinandergeklebt sind.

5. Medizinische Einrichtung nach Anspruch 1, wobei die Arrayfläche (430; 530; 630; 730) mehrere identisch konfigurierte Mikronadeln (432; 532; 632; 732) umfasst, die sich von der Arrayfläche erstrecken.

6. Medizinische Einrichtung nach Anspruch 5, ferner umfassend eine aktive Komponente, die auf mindestens einem Abschnitt der identisch konfigurierten Mikronadeln gehalten ist.

7. Medizinische Einrichtung nach Anspruch 1, wobei die Arrayfläche mehrere Mikronadeln umfasst, die sich von der Arrayfläche erstrecken.

8. Medizinische Einrichtung nach Anspruch 7, wobei die Mikronadeln sich verjüngende Strukturen umfassen, die mindestens einen Kanal enthalten, der in der Außenseitenfläche jeder Mikronadel ausgebildet ist.

9. Medizinische Einrichtung nach Anspruch 8, wobei die Mikronadeln längliche Basen umfassen und sich der mindestens eine Kanal in jeder Mikronadel von einem der Enden jeder länglichen Basis zu den Spitzen der Mikronadeln erstreckt.

10. Medizinische Einrichtung nach Anspruch 7, wobei die Mikronadeln ein Seitenverhältnis von 2:1 oder höher aufweisen.

11. Medizinische Einrichtung nach Anspruch 1, wobei das flexible Trägerglied (436; 536; 636; 736) ein Material ausgewählt aus der Gruppe bestehend aus Polyethylenterephthalat, Polycarbonat und Polyethylen umfasst.

12. Medizinische Einrichtung nach Anspruch 1, ferner umfassend eine Haftkleberschicht, die mindestens einen Abschnitt der ersten Hauptfläche des flexiblen Trägerglieds bedeckt.

## Revendications

1. Dispositif médical convenant pour être utilisé pour administrer un composant actif dans la peau ou à travers la peau et comprenant :
un élément de prolongation (422 ; 522 ; 622 ; 722) présentant une première surface principale (424 ; 524 ; 624 ; 724) et une deuxième surface principale (426 ; 526 ; 626 ; 726), la première surface principale comprenant une première partie et une deuxième partie flexible,
un élément (428 ; 528 ; 628 ; 728) de retenue de batterie s'étendant depuis la première partie de la première surface principale de l'élément de prolongation, l'élément de retenue de batterie comprenant une surface de batterie (430 ; 530 ;
630 ; 730) comportant au moins une microaiguille (432 ; 532 ; 632 ; 732) qui déborde de la surface de batterie,
un adhésif (434 ; 534 ; 634 ; 734) sensible à la pression disposé sur la deuxième partie de la première surface principale de l'élément de prolongation pour permettre la fixation par adhérence du dispositif sur la peau d'un mammifère lorsque la ou les microaiguilles sont insérées à travers le stratum comeum et
un élément flexible de dos (436 ; 536 ; 636 ; 736) qui présente une première surface principale (438 ; 538 ; 638 ; 738) et une deuxième surface principale (440 ; 540 ; 640 ; 740), au moins une partie de la première surface principale de l'élément flexible de dos étant fixée sur la deuxième surface principale de l'élément de prolongation, l'élément flexible de dos s'étendant au-delà du bord extérieur de l'élément de prolongation,
**caractérisé en ce que**
la deuxième surface principale de l'élément de prolongation est relativement lisse et uniforme.

2. Dispositif médical selon la revendication 1, dans lequel l'élément de prolongation (422 ; 522 ; 622 ; 722) et l'élément (428 ; 528 ; 628 ; 728) de retenue de batterie comprennent une structure en une pièce qui contient un matériau sélectionné dans l'ensemble constitué des polymères d'acrylonitrile, butadiène et styrène (ABS), des poly(sulfures de phényle), des polycarbonates, des polypropylènes, des acétals, des acryliques, des polyétherimides, des poly(téréphtalates de butylène), des poly(téréphtalates d'éthylène) et des combinaisons de deux ou plusieurs d'entre eux.

3. Dispositif médical selon la revendication 1, dans lequel l'élément de prolongation et l'élément de retenue de batterie contiennent du polycarbonate.

4. Dispositif médical selon la revendication 1, dans lequel l'élément de prolongation et l'élément de retenue de batterie sont retenus l'un sur l'autre par adhérence.

5. Dispositif médical selon la revendication 1, dans lequel la surface de batterie (430 ; 530 ; 630 ; 730) comprend plusieurs microaiguilles (432 ; 532 ; 632 ; 732) de configuration identique qui débordent de la surface de batterie.

6. Dispositif médical selon la revendication 5, comprenant en outre un composant actif retenu sur au moins une partie des microaiguilles de configuration identique.

7. Dispositif médical selon la revendication 1, dans lequel la surface de batterie comprend plusieurs microaiguilles qui débordent de la surface de batterie.

8. Dispositif médical selon la revendication 7, dans lequel les microaiguilles comprennent des structures effilées qui contiennent au moins un canal formé dans la surface extérieure de chaque microaiguille.

9. Dispositif médical selon la revendication 8, dans lequel les microaiguilles contiennent une base allongée et le ou les canaux ménagés dans chaque microaiguille débordent de l'une des extrémités de chaque base allongée jusqu'à la pointe des microaiguilles.

10. Dispositif médical selon la revendication 7, dans lequel les microaiguilles ont un rapport d'allongement de 2 : 1 ou supérieur.

11. Dispositif médical selon la revendication 1, dans lequel l'élément flexible de dos (436 ; 536 ; 636 ; 736) comprend un matériau sélectionné dans l'ensemble constitué du poly(téréphtalate d'éthylène), du polycarbonate et du polyéthylène.

12. Dispositif médical selon la revendication 1, comprenant en outre une couche d'adhésif sensible à la pression qui couvre au moins une partie de la première surface principale de l'élément flexible de dos.
